# EUROPEAN PATENT APPLICATION

(11) **EP 1 258 727 A1**
(43) Date of publication of application: **20.11.2002**
(21) Application number: 01902829.9
(22) Date of filing: 09.02.2001
(51) Int. Cl.: G01N 33/50, G01N 33/15, A61P 5/28

(54) **METHODS OF SCREENING FOR ANTI-ANDROGEN AGENTS**

(30) Priority: 10.02.2000 JP 2000038151
(71) Applicant: CHUGAI SEIYAKU KABUSHIKI KAISHA, Tokyo, 115-8543 (JP)
(72) Inventor: IKEDA, Kyoji, Nagoya-shi, Aichi 458-0014 (JP); NAKANISHI, Makoto, Nagoya-shi, Aichi 468-0023 (JP); KATO, Shigeaki, Tokorozawa-shi, Saitama 359-1121 (JP)
(74) Representative: VOSSIUS & PARTNER
(86) International application number: JP0100936
(87) International publication number: WO01059448

(57) **Abstract**

A novel action mechanism wherein androgen receptor (AR) is activated by the interaction between cyclin E and the androgen receptor was revealed. This activation is resistant to existing anti-androgen agents and both androgen receptor ligand-dependent and independent activations were found to participate in this mechanism. Methods of screening for anti-androgen agents using cyclin E are also provided by the present invention. Based on the findings as described above, novel anti-androgen agents can be screened. These anti-androgen agents are expected to be efficacious also against pathological conditions that have become resistant to existing anti-androgen agents. Thus, the instant invention opens up new possibilities for treatment using anti-androgen agents that had their limitations until now.

SEQUENCE LISTING

<110> CHUGAI SEIYAKU KABUSHIKI KAISHA

<120> METHODS OF SCREENING FOR ANTI-ANDROGEN AGENTS

<130> C1-108PCT

<140>
<141>

<150> JP 2000-038151
<151> 2000-02-10

<160> 1

<170> PatentIn Ver. 2.0

<210> 1
<211> 15
<212> DNA
<213> Artificial Sequence

<220>
<223> Description of Artificial Sequence: artificially
synthesized primer sequence

<220>

<221> misc_feature

<222> (7)..(9)

<223> "n" represents "a", "g", "c", or "t"

<400> 1

agaacannnt gttct                                                        15

## Description

### Technical Field

The present invention relates to methods of screening for compounds that inhibit interaction of cyclin E with androgen receptor (AR) , compounds that can be selected by the screening methods, and their therapeutic use.

### Background Art

Androgens are known to be highly involved in benign prostatic hyperplasia, male pattern baldness, precocious puberty, acne vulgaris, seborrhea, and hypertrichosis. Androgens also play a pivotal role in the development and progression of prostate cancer, one of the leading causes of death by cancer in men (Parker, S. L. et al., CA Cancer J. Clin. 46, 5-27 (1996)). Prostate cancers are initially androgen sensitive and respond well to androgen ablation therapy, which is widely used as a standard treatment for metastatic diseases (W.J. Catalona, N. Engl. J. Med. 331,996 (1994)).

As anti-androgen agents, in other words , antagonists of androgen receptor (AR), cyproterone acetate, chlormadinone acetate, flutamide, bicalutamide, and such, are used. Cyproterone acetate is known to repress progression of acne and development of baldness in teenagers. Cyproterone acetate is also used for treating female virilization and alopecia. Flutamide and bicalutamide are used as therapeutic agents for prostate cancer.

Hormone ablation therapy using these anti-androgen agents is effective in many cases including drug therapy of prostate cancer, and therefore, these agents have become effective therapeutic agents. However, one problem is that, in most cases, tumor recurrence ensues within two to five years following even an effective treatment with an anti-androgen agent. In other words, the tumor becomes resistant to the anti-androgen agent. The molecular mechanism underlying the resistance to hormone ablation therapy remains largely unknown.

Androgens and estrogens regulate the growth of hormone-responsive prostate and breast cancer through androgen receptor (AR) and estrogen receptors , respectively, which belong to the nuclear receptor superfamily (Mangelsdorf, D.J. et al., Cell 83, 835-839 (1995)). Mutations in AR genes have been reported in metastatic hormone-independent prostate cancer (Taplin, M.E. et al., N. Engl. J. Med. 332, 1393-1398 (1995)), which may account for the altered response of androgen receptor (AR) to hormonal environment. However, this is a relatively rare event, and does not provide a general clue to the adaptive response of most prostate tumors to endocrine therapy. A recent experiment in which surgical samples were transferred to SCID mice has shown that androgen-independent, metastatic tumors can restore hormone responsiveness in a different host (Klein, K.A. et al., Nat. Med. 3, 402-408 (1997)), suggesting that exogenous cellular factor(s) , not the AR itself, are involved in the regulation of AR functions in the nucleus.

The cell division inducing signal of androgens is considered to ultimately influence cell cycle machinery. During the progression from the GI to S phase of the cell cycle, successive and coordinated activation of cyclin-dependent kinases (Cdks) takes place, whose activities are positively regulated by cyclin subunits and negatively by Cdk inhibitors (Hartwell, L. H. & Kastan, M. B., Science 266,1821-1828 (1994); Sherr, C. J., Science 274,1672-1677 (1996); Hunter, T. & Pines, J., Cell 79,573 (1994)) . It has been shown that estrogens (Prall, 0. W. et al., J. Steroid Biochem. Mol. Biol. 65, 169-174 (1998); Planas-Siva, M. D. & Weinberg, R. A., Mol. Cell. Biol. 17, 4059-4069(1997)) and androgens (Lu, S. et al., Cancer Res. 57, 4511-4516 (1997); Menjo, M. et al., Oncogene 17, 2619-2627 (1998); Kokontis, J. M. et al., Mol. Endocrinol. 12, 941-953 (1998)) regulate Cdk activities by modulating the expression of cyclins and /or Cdk inhibitors and thereby stimulate the G1-S transition. In addition, evidence for the direct binding of ER to cyclin D1 has been provided (Zwijsen, R. M. L. et al., Cell 88, 405-415 (1997); Neuman, E. et al., Mol.Cell. Biol. 17, 5338-5347 (1997)). In view of the frequent overexpression and amplification of cyclin D1 gene in breast cancer (Buckley, M. F. et al. , Oncogene 8, 2127-2133 (1993); Keyomarsi, K. & Pardee, A. B., Proc. Natl. Acad. Sci. USA 90, 1112-1116 (1993)), it is suggested that cyclin D1 may play a role in Cdk-independent activation of estrogen receptor in breast tumors. However, little is known as to whether AR binds directly to components of the cell cycle machinery and how this interaction modulates the transcriptional activity of AR in prostate cancer.

### Disclosure of the Invention

The present invention is based on the finding of the present inventors and others that cyclin E, a constituent of the cell cycle mechanism, enhances transcriptional activation of androgen receptor (AR) by binding thereto.

More specifically, the present invention provides methods of screening for compounds that inhibit interaction of cyclin E with androgen receptor (AR). The present invention also provides compounds that can be isolated by screening and their therapeutic use; especially as novel anti-androgen agents for preventing and treating a disease in which androgen is involved, including prostate cancer.

Although androgens play a pivotal role in the growth of prostate cancer as above, the molecular mechanism underlying resistance to anti-androgen therapy remains unknown. In the present study, the present inventors studied the functional and physical interaction between cyclin E and AR.

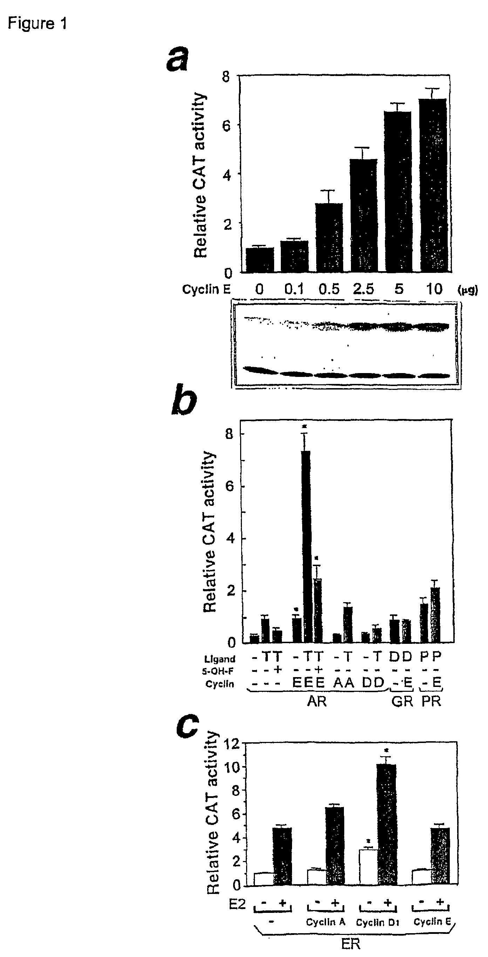

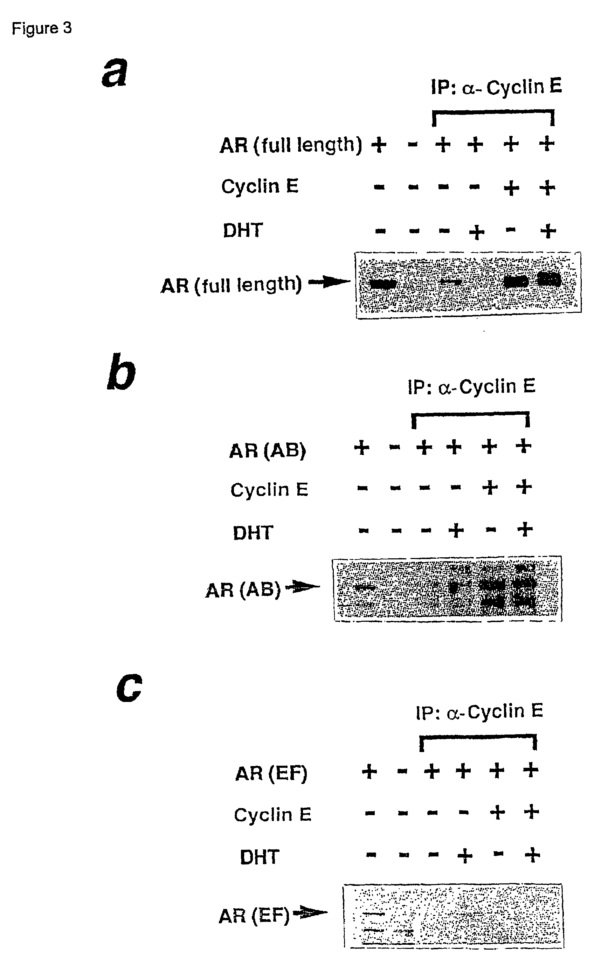

First, effects of cyclin on AR activity were investigated by transiently expressing AR and cyclin in AR-negativecells. As a result, the present inventors found that cyclin E (Published Japanese Translation of International Publication No. Hei 7-502164) enhanced both ligand-independent and -dependent transcriptional activity of AR, and that these activities were not sufficiently inhibited by the anti-androgen agent, 5-hydroxyflutamide (Figure 1). It was found that cyclin E bound directly to the C-terminal portion of the AB domain of AR (Fig. 3). In addition, using co-immunoprecipitation and GST pull-down assay, the inventors found that cyclin E directly bound to the AB domain of AR independently of the binding of Cdk2 or hormones (data not shown). These results suggest that cyclin E functions as a co-activator of AR and that aberrant expression of cyclin E in tumors may lead to persistent activation of AR function even under androgen ablation therapy. These results suggest that the onset of the resistance of prostate cancer to hormone ablation therapy may be based on inappropriate activation of AR by cyclin E.

Therefore, the provision of compounds that interfere with the interaction between AR and cyclin E may provide a novel and attractive strategy in the prevention and treatment of diseases associated with androgen, including prostate cancer.

The present invention provides methods of screening for compounds that inhibit interaction between cyclin E and androgen receptor (AR) , compounds that can be selected by the screening, and their therapeutic use, which are expected to be applied as therapeutic agents for these novel therapeutic strategies.

More specifically, the present invention provides the following:
(1) a method of screening for a compound that inhibits the interaction of cyclin E with androgen receptor, comprising the steps of:
   (a) contacting cyclin E and the androgen receptor in the presence of a test sample,
   (b) detecting the binding of cyclin E to the androgen receptor, and
   (c) selecting a compound that decreases the binding compared to when it is detected in the absence of the test sample;
(2) a method of screening for a compound that inhibits interaction of cyclin E with androgen receptor, comprising the steps of:
   (a) contacting a test sample with a cell into which a vector expressing cyclin E, a vector expressing androgen receptor, and a vector in which a reporter gene is operably linked to the downstream of androgen receptor responsive element, have been introduced,
   (b) detecting the reporter activity in the cell, and,
   (c) selecting a compound that decreases the reporter activity compared to when it is detected without contacting the test sample;
(3) a compound that inhibits the interaction of cyclin E with androgen receptor, a pharmaceutically acceptable salt thereof, or a prodrug thereof;
(4) the compound, pharmaceutically acceptable salt, or prodrug of (3), selected by the method of (1) or (2);
(5) a pharmaceutical composition comprising the compound, pharmaceutically acceptable salt, or prodrug of (3) or (4) as the active ingredient;
(6) the pharmaceutical composition of (5) , which is an anti-androgen agent; and
(7) the pharmaceutical composition of (5) or (6) , which is used for preventing and treating diseases selected from the group consisting of prostate cancer, benign prostatic hyperplasia, male pattern baldness, precocious puberty, acne vulgaris, seborrhea, and hypertrichosis.

The present invention provides methods of screening for compounds that inhibit interaction of cyclin E with AR. One embodiment of the screening methods of this invention is a method using the inhibition of binding activity of cyclin E to AR as an index. One embodiment of this method comprises the steps of (a) contacting cyclin E and the androgen receptor (AR) in the presence of a test sample, (b) detecting the binding of cyclin E to the androgen receptor (AR) , and (c) selecting a compound that decreases the binding compared to when it is detected in the absence of the test sample.

Cyclin E and AR used in this screening can be recombinant or natural proteins. "Cyclin E" includes not only the complete protein of cyclin E, but also a partial peptide thereof as long as it has the activity to bind to AR. "AR" includes not only the complete protein, but also a partial peptide thereof as long as it has the activity to bind to cyclin E. For example, since the AB domain of AR was shown to be directly bound to cyclin E, a peptide including AB domain of AR can be suitable for this screening. AB domain (amino acid residues 1 to 544) and EF domain (amino acid residues 624 to 918) of cyclin E is described in Doesburg, P. et al., Biochemistry 36, 1052-1064 (1997), Elizabeth, L. et al., J. Biol. Chem. 270, 29983-29990 (1995).

"Contacting" and "binding" of this invention includes not only direct "contacting" and "binding", but also indirect "contacting" and "binding". For example, this screening is possible by using a partial peptide of cyclin E even if the peptide is bound to AR indirectly through another protein factor.

Cyclin E and AR can be used, for example, in the form bound to a carrier or as fusion protein linked to another protein depending on the method of screening described below.

Samples tested include, but are not limited to, cell extracts, cell culture supernatants, products of fermentation microorganisms , marine organism extracts, plant extracts, purified or crude preparations of proteins, peptides, non-peptide compounds, synthetic low-molecular weight compounds, natural compounds, and gene libraries. Cyclin E can be contacted with AR either *in vitro* or in vivo depending on the method of screening described below.

An example of a method of screening is specifically shown below.

The screening of this invention can be conducted by pull down assay, etc. Cyclin E is contacted with AR in the presence or absence of a test sample. Then, Cyclin E-AR complex is recovered using an antibody against cyclin E or AR, or such, and the binding is measured. Proteins can be fusion proteins made with other peptides or other proteins as mentioned above. When an antibody against cyclin E or AR is used, the complex can be precipitated using Protein A Sepharose and Protein G Sepharose. When cyclin E or AR is prepared as a fusion protein with an epitope such as GST, the complex can be recovered using a material that can specifically bind to an epitope such as glutathione-Sepharose 4B, as in the case when an antibody is used against cyclin E and AR.

Cyclin E or AR can be labeled suitably. Labelingmethods include a method that utilizes the binding between biotin and avidin, or a method that utilizes an antibody that specifically binds to cyclin E, AR, or a peptide or polypeptide (e.g. GST and such) that is fused thereto. Methods using radioisotope or fluorescence, and such, may also be used.

In the pull down assay, purified cyclin E and AR can be contacted not only *in vitro,* but also *in vivo*.

In the in vivo method, for example, a vector expressing exogenous cyclin E and AR is introduced into a cell, and the cell is contacted with a test sample. Thereafter, a lysate of the cell is prepared. This lysate is contacted with an antibody against cyclin E or AR, or such, and the formed complex is precipitated.

A cell into which a vector expressing endogenous cyclin E and AR, but not exogenous cyclin E and AR, is introduced can also be contacted with a test sample and analyzed in the same way.

Moreover, screenings of this invention can be conducted by, for example, via affinity chromatography. For example, either cyclin E or AR protein is fixed onto the carrier of the affinity column, and another protein is applied onto the column and bound to it. Then, a test sample is applied and a compound that inhibits the binding of cyclin E to AR is screened. The screening can be done using a microplate or such to which protein is fixed.

A biosensor utilizing the surface plasmon resonance phenomenon may be used as a means for detecting or measuring a compound bound to the present invention. When such a biosensor is used, the interaction between cyclin E and AR can be observed at real-time as a surface plasmon resonance signal without labeling (for example, BIAcore, Pharmacia). Therefore, it is possible to evaluate the binding between cyclin E and AR using a biosensor such as BIAcore.

Screenings of this invention can also be conducted using the two-hybrid system (Fields, S. and Sternglanz, R., Trends. Genet. 10, 286-292 (1994); Dalton, S. and Treisman, R., Characterization of SAP-1, a protein recruited by serum response factor to the c-fos serum response element., Cell 68, 597-612 (1992); "MATCHMAKER Two-Hybrid System", "Mammalian MATCHMAKER Two-Hybrid Assay Kit", "MATCHMAKER One-Hybrid System" (all Clontech), "HybriZAP Two-Hybrid Vector System" (Stratagene)) . For example, in the yeast two-hybrid system, either cyclin E or AR protein is fused with a peptide that binds to a specific DNA sequence such as SRF DNA binding region or GAL4 DNA binding region. On the other hand, another protein is fused with a peptide that activates transcription such as VP16 or GAL4 transcription activating region. Both fusion proteins are expressed in a yeast cell. A positive clone can be confirmed in the absence of a test sample as the reporter gene is activated by the binding of cyclin E to AR. In the presence of the test sample, the same experiment is performed, and a compound that decreases the expression of the reporter gene is selected. For example, Ade2 gene, LacZ gene, CAT gene, luciferase gene, PAI-1 (Plasminogen activator inhibitor type1) gene, and so on, can be mentioned as reporter genes used in the two-hybrid system in addition to the HIS3 gene, but reporter genes are not restricted thereto. Not only yeast cells, but also mammalian cells as described in the examples can be used suitably for the two-hybrid system.

Cyclin E and AR used for the screening are not specially limited as long as both show binding activities. For example, a peptide comprising AB domain of AR is a preferable example since AB domain of AR was shown to bind directly-to cyclin E. Cells such as COS cells, HeLa cells, CHO cells, and MDAH041 cells can be used as mammalian cells. However, many other cells can also be employed, as there are no special conditions restricting cells that can be used.

As a result of the screening, if the expression of the reporter gene is significantly decreased by a test compound compared to a control in which the test sample is not contacted or introduced, the test compound used is a candidate for a compound that inhibits the interaction of cyclin E with androgen receptor (AR).

In the screenings of this invention, a reporter system can also be used to increase the efficiency of screening. That is, another embodiment of the present invention's screening for a compound that inhibits the interaction of cyclin E with androgen receptor (AR) can be conducted by a method comprising the steps of (a) contacting a test sample with a cell into which a vector expressing cyclin E, a vector expressing androgen receptor (AR), and a vector in which a reporter gene is operably linked to the downstream of androgen receptor responsive element, have been introduced, (b) detecting the reporter activity in the cell, and (c) selecting a compound that decreases the reporter activity compared to when it is detected without contacting the test sample.

Expression vectors of cyclin E and androgen receptor (AR) can be constructed, for example, by integrating cyclin E gene, androgen receptor gene, and such, into mammalian expression vectors such as pSG5 and pcDNA3. In addition, vectors in which a reporter gene is operably linked to the downstream of androgen receptor responsive element can be constructed by integrating ARE or HRE into the expression vector into which the reporter gene is integrated. As ARE sequence, for example "5'-AGAACANNNTGTTCT-3' " (SEQ ID NO: 1) which is a consensus sequence of AR/GR/PR/MR can be used. As reporter gene, for example, the CAT gene, luciferase gene and so on can be used, but is not limited thereto. As cells used for the screenings, for example, COS7 cells, COS1 cells, HeLa cells, MDAH041 cells, PC-3 cells, LNCaP cells, DU-145 cells, etc., can be used.

As a result of measuring the expression of the reporter gene, if the detected reporter gene activity is significantly decreased by the contact or introduction of the test sample compared to a control in which the test sample is not contacted or introduced, the test compound used is a candidate for a compound that inhibits the interaction of cyclin E with the androgen receptor (AR). The screening systems of this invention enable the screening of inhibitors of AR activation, which is resistant to known androgen inhibitors.

By above-mentioned screenings of this invention, compounds having the activity to inhibit the interaction between cyclin E and androgen receptor (AR) can be isolated. The compounds obtained by using the screening methods of this invention include compounds whose structure is partially altered by an addition, deletion, and/or substitution.

A compound that inhibits the interaction between cyclin E and AR can be a candidate for an anti-androgen agent. For example, it is expected to be applied to the therapy of various diseases in which AR is involved, such as prostate cancer, benign prostatic hyperplasia , male pattern baldness, precocious puberty, acne vulgaris, seborrhea, and hypertrichosis. Furthermore, the compound can be expected to prevent or delay development of diseases such as prostate cancer, benign prostatic hyperplasia, male pattern baldness, precocious puberty, acne vulgaris, seborrhea, and hypertrichosis if it is administered in advance. Thus, it is expected to be applied as a preventive drug for these diseases.

In addition to the reporter assay using AR gene as described in the examples, the evaluation of whether a compound obtained by a screening has an anti-androgen effect or not can be done more precisely by, for example, suitably combining the assays below, if desired.

### <The assay of an antagonist action by an in vivo experiment in rat>

When testosterone or dihydrotestosterone is administered to castrated rats, the weight of prostate and seminal vesicle increase. The antagonist effect of a test compound can be measured by examining whether or not the test compound represses the above weight increasing effect due to the administration of testosterone or dihydrotestosterone. Measurement can be made according to the methods disclosed in J. Med. Chem. 41: 623-639, 1998 and Kiso-to-Rinsho 29(4): 877-885, 19-95.

When using a compound of this invention, which inhibits the interaction between cyclin E and AR, or a pharmaceutically acceptable salt of the compound, or a prodrug thereof, as a pharmaceutical agent for humans and other mammals , such as mice, rats , guinea-pigs , rabbits , cats, dogs, sheep, pigs, cattle, monkeys, baboons, and chimpanzees, and also for chicken, the compound itself can be directly administered to the patient or can be formulated for administration using known methods for preparing pharmaceuticals. The pharmaceutical compositions of this invention, whose active ingredients are compounds that inhibit the interaction between cyclin E and androgen receptor (AR), can be taken orally, for example, as sugarcoated tablets, capsules, elixirs, and microcapsules, or non-orally in the form of injections of sterile solutions or suspensions with water or any other pharmaceutically acceptable liquid, according to the need. For example, the compounds can be formulated by suitably mixing with pharmacologically acceptable carriers or media; specifically, sterilized water, physiological saline, plant-oil, emulsifiers, solvents, surfactants, stabilizers, flavoring agents, excipients, vehicles, preservatives, and binders, etc., in a unit dosage form required for generally acceptable drug implementation. The amount of active ingredients in these preparations makes a suitable dosage within the indicated range acquirable.

Examples of additives that can be mixed to tablets and capsules are, binders such as gelatin, corn starch, tragacanth gum, and arabic gum; excipients such as crystalline cellulose; swelling agents such as corn starch, gelatin, and alginic acid; lubricants such as magnesium stearate; sweeteners such as sucrose, lactose, or saccharin; flavoring agents such as peppermint, Gaultheria adenothrix, oil, and cherry. When the unit dosage form is a capsule, a liquid carrier, such as oil, can also be included in the above ingredients. Sterile composites for injections can be formulated following normal drug implementation methods using vehicles such as distilled water used for injections.

Physiological saline, glucose, and other isotonic liquids including adjuvants, such as D-sorbitol, D-mannose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Sesame oil or Soy-bean oil can be used as a oleaginous liquid and may be used in conjunction with benzyl benzoate or benzyl alcohol as a solubilizer; may be formulated with a buffer such as phosphate buffer and sodium acetate buffer; a pain-killer such as procaine hydrochloride; a stabilizer such as benzyl alcohol, phenol; and an anti-oxidant. The prepared injection is usually filled into a suitable ampule.

Although the content of a compound that inhibits the interaction between cyclin E and AR in such a preparation varies according to its formulation type, it is preferably, 5 to 100 weight% in general.

Methods well known to one skilled in the art may be used to administer a pharmaceutical composition to patients. For example, it can be given as intra-arterial, intravenous, or subcutaneous injections, intranasal, transbronchial, intramuscular, percutaneous, or via oral administrations. The dosage varies according to the body-weight and age of a patient, the administration method, and such, but one skilled in the art can suitably select an appropriate dosage. If a DNA can encode the compound, the DNA can be inserted into a vector for gene therapy to perform the therapy. The dosage and method of administration vary according to the body-weight, age, and symptoms of a patient, but one skilled in the art can select them suitably.

The dosage of a compound of this invention, which inhibits the interaction between cyclin E and AR, a pharmaceutically acceptable salt thereof, or a prodrug thereof, can vary widely depending on the type of warm-blooded animal (including humans) to which the compound is given, the extent of symptoms, the doctor's diagnosis, etc. Generally, the dosage of the active ingredient is 1 µg-500 mg/kg a day, and preferably 20 µg-100 mg/kg a day. Moreover, the above-mentioned dosage can be administered once or several times, per day or per month. It can be changed based on the extent of symptoms , and the doctor's diagnosis.

### Brief Description of the Drawings

Figure 1 is a diagram and photograph showing that cyclin E enhances the transcriptional activity of AR.
a, AR-negative MDAH041 cells were co-transfected with an expression vector for AR (1 µg), an ARE-CAT reporter construct (8 µg) , and various amounts of an expression vector encoding cyclin E. After cells were cultured in the presence of 10⁻⁹ M dihydrotestosterone for 18 h, CAT assays were performed.
b, MDAH041 cells were co-transfected with an expression vector encoding AR, GR, or PR (1 µg) , an ARE-CAT reporter construct (8 µg) and an expression vector encoding cyclin E, cyclin A, or cyclin D1 (8 µg each). After cells were cultured in the presence of 10⁻⁹ M dihydrotestosterone ("T" in the figure), dexamethasone ("D" in the figure), or progesterone ("P" in the figure) for 18 h, CAT assays were performed with the extracts from the cells. Some cultures received 10⁻⁵ M 5-hydroxy-flutamide (5-OH-F "+" in the figure).
c, MDAH041 cells were transfected with ER expression vector (1 µg) and ERE-CAT reporter construct (8 µg) in the absence or presence of expression vectors encoding cyclin A, cyclin D1, or cyclin E (8 µg each) , and CAT assays were performed with extracts from the cells cultured for 18 h in the absence (-) or presence (+) of 17β-estradiol (E₂, 10⁻⁸ M).

All transfections described above (i.e., a-c) included β-galactosidase expression vector (3 µg) as an internal control for measuring differences in transfection efficiency. CAT activity was corrected with β-galactosidase activity.

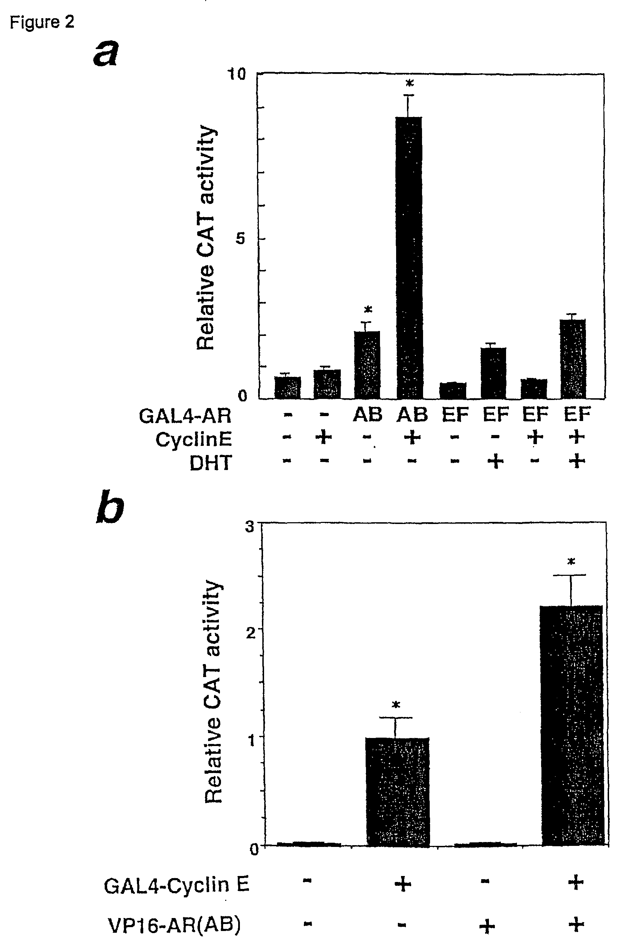

Figure 2 is a diagram showing the functional interaction of cyclin E with AB domain of AR.
a, Interaction of cyclin E with AB or EF domain of AR was examined in the mammalian two-hybrid system. HeLa cells were co-transfected with a CAT reporter construct containing GAL4 binding element, expression vector encoding AB or EF domain of AR fused to GAL4 DNA-binding domain (GAL4-AR), and an expression vector for cyclin E. Some cultures were treated with dihydrotestosterone (DHT "T" in the figure) at 10⁻⁹ M for 18 h and then CAT assays were performed.
b, InteractionbetweenGAL4-cyclin E and VP16-AR(AB) was examined in a similar manner.

Figure 3 is a diagram and photograph showing that cyclin E binds to AR in vivo.

MDAH041 cells transfected with expression vectors for cyclin E and full-length AR (a) , AB (b) , or EF region of AR (c) were cultured in the absence (-) or presence (+) of dihydrotestosterone (DHT, 10⁻⁹ M) . Cells were lysed in TNE buffer, and AR (a) , AR(AB) (b) , or AR(EF) (c) was detected in the anti-cyclin E immunoprecipitates with an antibody specific to AB (a,b) or EF (c) domain of AR, respectively.

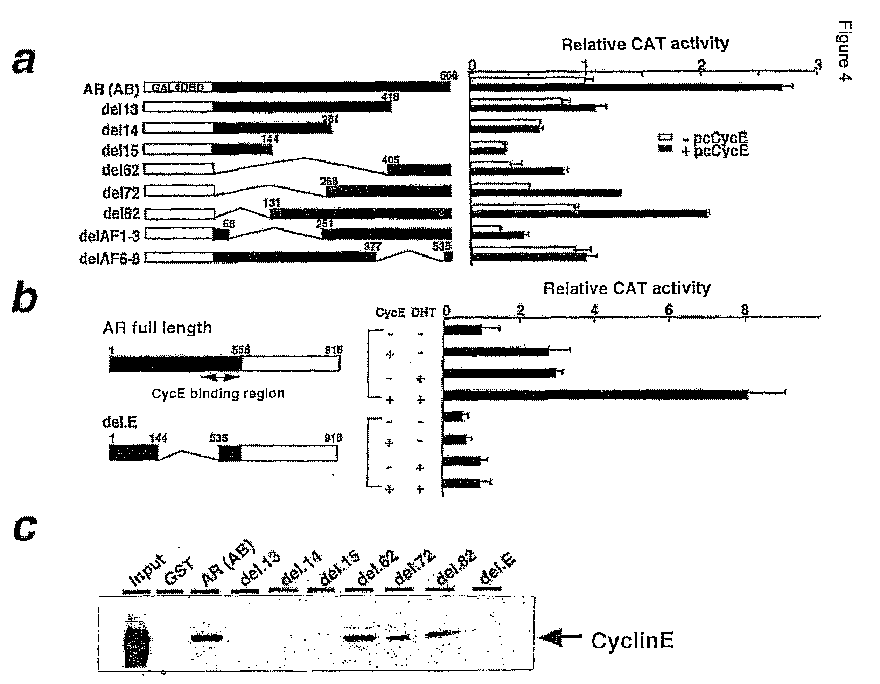

Figure 4 is a diagram and photograph showing localization of cyclin E-binding domain within AB domain of AR.
a, HeLa cells were co-transfected with a CAT reporter construct containing GAL4 binding element, an expression vector encoding AR (AB) or its deletion mutants fused to GAL4 DNA-binding domain (shown to the left), in the absence (open bars) or presence (closed bars) of an expression vector for cyclin E.
b, Full-length AR or AR devoid of both cyclin E-binding and transactivation domains (del.E) was co-transfected into HeLa cells with an ARE-CAT reporter construct in the absence or presence of cyclin E expression vector, and CAT activity was determined after cells were cultured with or without 10⁻⁹ M dihydrotestosterone for 18 h.
c, shows that Cyclin E binds directly to the C-terminal portion of AR(AB) in vitro. GST-fused AR (AB) and its deletion proteins were incubated with in vitro translated cyclin E. The complexes were adsorbed to glutathione beads, and bound proteins were analyzed on SDS-PAGE followed by autoradiography.

### Best Mode for Carrying out the Invention

The present invention will be explained in detail below with reference to examples, but it is not to be construed as being limited thereto. All the references cited herein are incorporated into the description as a part of this invention.

### [Example 1] Cyclin E enhances transcriptional activity of androgen receptor (AR)

In order to examine whether functional cross talk exists between AR and cell cycle regulators, AR-negative human fibroblastic MDAH041 cells (Nakanishi M. et al. (1995) EMBO J. 14:555-563) were co-transfected with an AR expression vector, ARE-CAT reporter construct, and an expression vector encoding cyclin D1, cyclin E, or cyclin A, and the transactivation function of AR through ARE was determined as follows in the absence or presence of its ligand, dihydrotestosterone (DHT).

Full-length human cyclin E, cyclin D1, and cyclin A were inserted into the pcDNA3 vector. Full-length human AR, estrogen receptor α (ERα) , glucocorticoid receptor (GR) , and progesterone receptor (PR) were inserted into the mammalian expression vector pSG5, pSG1, pKCR2 and pSG5, respectively. The reporter constructs pARE2tk-CAT, pGRE-tk-CAT, pPRE-tk-CAT, and pBL-CAT8+/ERE have been described previously (Tsai, S.Y. et al. (1989) Cell 57: 443-448).

HeLa and MDAH041 cells were maintained in Dulbecco's modified Eagle medium (DMEM) supplemented with 10% fetal bovine serum (FBS). Twenty-four hours before transfection, the medium was changed to DMEM without phenol red containing 5% activated charcoal-treated FBS. Cells were transfected with 8 µg of ARE-CAT reporter construct (pARE2tk-CAT) , 3 µg of β-galactosidase expression vector (as an internal control to determine transfection efficiency) , 1 µg of AR expression vector and 8 µg of cyclin E, cyclin D1, or cyclin A expression vector, using the calcium phosphate precipitation technique. Eighteen hours after transfection, cells were rinsed in phosphate-buffered saline (PBS) and re-fed with fresh medium containing ligands or vehicles. In some cases, ligand (testosterone or dihydrotestosterone (DHT)) was added. Eighteen hours later, cells were harvested and assayed for CAT and β-galactosidase activities. CAT activity was corrected with β-galactosidase activity (Fig. 1). In the experiment described later (Fig. 2a) , GR or PR expression vectors were used instead of the AR expression vector, and dexamethasone and progesterone were used as ligands, respectively. At this time, pGRE-tk-CAT and pPRE-tk-CAT were used as reporter plasmids for GR and PR, respectively. Furthermore, the effect of estrogen (17β-estradiol; 10⁻⁸ M) was also examined using ER expression vector and pBL-CAT8+/ERE as a reporter plasmid.

Figure 1a shows that cyclin E enhanced the transcriptional response of AR to DHT, depending on the amount of cyclin E expression vector. Cyclin E did not increase ARE-mediated transcription without co-transfection of AR (data not shown) , indicating that cyclin E acts through AR. It was found that the effect on AR-mediated transcription was specific to cyclin E since cyclin A had no effect and cyclin D1 caused a slight suppression of AR transcriptional activity (Fig. 1b) . In these experiments, the level of AR protein did not increase by overexpression of cyclin E (data not shown) , indicating that cyclin E enhances AR function, without altering its expression level. Addition of an expression vector encoding Cdk2 in combination with cyclin E did not further enhance the ARE-mediated transcription (data not shown) , which is consistent with the notion that complex formation of cyclin E with Cdk2 is not required and that free cyclin E, rather than cyclin E/Cdk2 complex, is involved in the activation of AR.

Importantly, DHT-induced activation of the ARE-CAT reporter construct was inhibited totally by an anti-androgen agent, 5-hydroxyflutamide (5-OH-F), where as when cyclin E was co-transfected, AR function, was not completely suppressed even by a 1000 molar excess of 5-OH-F, and a substantial CAT activity remained (Fig. 1b).

Although glucocorticoid receptor (GR) and progesterone receptor (PR) are known to act through the same DNA element as AR ( Cato,A. et al. EMBO J. 7: 1403-1440 (1988)), it was found that the potentiation of ARE-mediated transcription by cyclin E was specific to AR signaling, since cyclin E failed to augment the transcriptional activation through GR or PR in response to dexamethasone or progesterone, respectively (Fig. 1b). When human estrogen receptor α (ERα) was introduced into MDAH041 cells, they responded to 17β-estradiol (E₂) with increased CAT activity (Fig. 1c). Cyclin E did not affect estrogen-induced transactivation function of ER through ERE, while cyclin D1 caused an increase in ERα function (Fig. 1c), as reported previously (Zwijsen, R. M. et al., Cell 88: 405-15, 1997; Neuman, E. et al., Mol. Cell. Biol. 17: 5338-47, 1997). The results shown in Fig. 1 have also been seen in HeLa cells (data not shown).

### [Example 2] Cyclin E mainly activates AF-1 function of AR

Like ER, AR has two transc-riptional activation functions, ligand-independent (AF-1) and dependent (AF-2) activation domains, which are located in the amino-terminal AB region and in the carboxyl-terminal ligand-binding EF domain, respectively (Langley, E. et al. (1995) J. Biol. Chem. 270: 29983-29990, Doesburg, P. et al. (1997) Biochemistry 36: 1052-1064). In order to localize the domain of AR that interacts with VP16-cyclin E fusion protein or cyclin E alone and which is responsible for the enhanced transcription activity, either AB or EF region of AR was fused to the GAL4 DNA binding domain (DBD), and functional interaction with VP16-cyclin E fusion protein or cyclin E alone was studied in the mammalian two-hybrid system with a GAL4-dependent reporter.

For mammalian two-hybrid assay, GAL4-AR(AB) and GAL4-AR(EF) were constructed in pM vector and VP16-cyclin E in pVP16 vector (CLONTECH Laboratories, Inc.), respectively (AB domain: amino acid residues 1-544, EF domain: amino acid residue 624-918). 17M2-G-CAT and 17M5-TATA-CAT reporter plasmids contain two GAL4 17M binding sites located upstream of the globulin promoter and the minimal promoter region of the adenovirus-2 major late promoter, respectively.

HeLa and MDAH041 cells maintained in Dulbecco's modified Eagle medium (DMEM) supplemented with 10% fetal bovine serum (FBS) were used in the assay. MDAH041 or HeLa cells were co-transfected with 2 µg GAL4-AR (AB) or GAL4-AR (EF) expression vector, 4 µg VP16-cyclin E, and 8 µg 17M₂-G-CAT reporter gene, and cultured in the absence orpresence of dihydrotestosterone (10⁻⁸ M). CAT assays were performed as described above.

As shown in Fig. 2a, GAL4-AR (AB) alone exhibited a constitutive activity even without the ligand, due to its AF1 function, and co-transfection of GAL4-AR (AB) with either VP16-cyclin E (data not shown) or cyclin E alone (Fig. 2a) further increased CAT activity.

However, a significant interaction of AR(EF) with cyclin E was not seen in the presence of DHT (Fig. 2a). Taken together with the results that cyclin E itself, or cyclin E fusion protein with GAL4DBD, is capable of stimulating transcription (Fig. 2b), it is suggested that cyclin E not only interacts with the AB domain of AR, but also functions as a cofactor of AR.

### [Example 3] Cyclin E binds directly to AB domain of AR

In order to obtain evidence for physical association between cyclin E and AR *in vivo,* AR-negative MDAH041 cells were transfected with AR alone or AR plus expression vector encoding cyclin E, cyclin D1, or cyclin A. After cyclin complexes were immunoprecipitated with antibody specific to respective cyclin subunit, cyclin-bound AR was detected by Western blotting using anti-AR antibody.

First, MDAH041 cells were transfected with expression vectors encoding cyclin E and full-length AR, AB domain, or EF domain of AR, and cultured in the absence or presence of testosterone. Cells were lysed in IP buffer containing 150 mM NaCl, 2.5 mM EGTA, 1 mM EDTA, 0.1% Tween-20, 10% glycerol, 50 mM HEPES, pH 8.0, and protease inhibitors (2 µg/ml of leupeptin, 100 µg/ml of PMSF, 2 µg/ml of aprotinin, and 20 µg/ml of trypsin inhibitor). The cell lysates were assayed for protein concentration by Bradford method (Bio-rad Laboratories, Richmond, CA), and equal amounts of proteins were used for immunoprecipitation or Western analysis.

For immunoprecipitations, cell lysates were incubated with anti-cyclin E antibody (Santa Cruz Biotechnology) and then with protein A agarose beads (Boehringer Mannheim, Mannheim, Germany) at 4°C. After 1 hr, beads were collected by brief centrifugation and boiled in Laemmli buffer. Samples were separated on a 10% SDS/polyacrylamide gel and transferred to nitrocellulose membrane. After blocking with PBS containing 5% milk and 0.1% Tween 20, proteins were detected with polyclonal antibody against human AR (Santa Cruz Biotechnology) and peroxidase-conjugated goat anti-mouse IgG. The blots were washed in PBS containing 0.1% Tween 20 and developed by enhanced chemiluminescence (ECL) reactions (Amersham).

As shown in Fig. 3a, when cells were co-transfected with expression vectors for full-length AR and cyclin E, an intense band of AR was detected in the anti-cyclin E immunoprecipitates, and the band became broader following treatment with DHT. A faint band of AR was also detected in the anti-cyclin E immunoprecipitates from the cells transfected with AR alone, which presumably reflects the association between endogenous cyclin E and transfected AR. Again, treatment with DHT produced a broader band of AR, which is likely to reflect phosphorylation in a subset of AR (Prall, 0.W. et al., J. Steroid Biochem. Mol. Biol. 65, 169-174 (1998); Blok, L. J. et al., Endocr. Res. 3, 197-219 (1996)). In these transfectants, the expression level of AR protein was comparable (data not shown) ; ruling out the possibility that overexpression of cyclin E results in an increased AR level and thereby stimulates ARE-mediated transcription. AR was not detected in the anti-cyclin A or anti-cyclin D1 immunoprecipitates whether in the absence or presence of DHT (data not shown) , indicating that the binding of AR is specific to cyclin E in vivo.

In order to gain insight into which region of AR physically interacts with cyclin E *in vivo,* MDAH041 cells were co-transfected with expression vector for cyclin E and either N-terminal AB or C-terminal EF region of AR, and the anti-cyclin E immunoprecipitates were assessed for the co-precipitation of respective AR fragment using antibody specific to either N-terminal or C-terminal region of AR, respectively. The N-terminal AB domain of AR (amino acids 1 to 554) and C-terminal EF domain (amino acids 624 to 918) were cloned into the pGEX(4T-1) vector and used for the experiments.

As shown in Fig. 3b, AB domain of AR was co-immunoprecipitated with cyclin E, whereas EF domain of AR was not (Fig. 3c) , indicating that cyclin E associates mainly with AB domain of AR independently of ligand binding.

Finally, the inventors examined via GST pull down assay whether cyclin E binds directly to AR (AB) and, if so, which region of AR(AB) it binds to.

GST protein, GST-AR(AB) fusion protein, and fusionproteins with GST and various mutants having deletions in a portion of the AB domain were produced in *Escherichia coli* and purified using glutathione-Sepharose beads (Pharmacia) as described previously (Nakanishi, M. et al. (1995) EMBO J. 14: 555-563, Hashimoto, Y. et al. (1998) J. Biol. Chem. 273: 16544-16550; Yanagisawa, J. et al. Science 283, 1317-1321 (1999)).

Cyclin E protein was translated in vitro in the presence of [³⁵S] methionine with the reticulocyte lysate system (Promega). Labeled cyclin E protein was incubated with GST, GST-fused AR (AB) , or partial amino acid deletion mutants for 1 hr and the mixture was incubated with glutathione beads for an additional 1 h. Bound proteins were then analyzed by SDS-PAGE and the signals were detected by autoradiography.

GST-fused AR(AB) or various deletion mutant proteins (del 13 - 15 and 62 - 82 in Fig. 4a) were mixed with *in vitro* translated cyclin E.

A GST pull-down assay revealed that cyclin E directly binds to the AB domain of AR (Fig. 4c), but only barely to the EF region of AR (data not shown) . Cyclin E bound to N-terminally truncated mutant proteins of AR(AB), such as del 62, del 72 and del 82, but not to C-terminally truncated mutants, including del 13, del 14 and del 15 (Fig. 4c), indicating that the cyclin E-binding domain resides in the C-terminal portion of AR(AB) (Fig. 4b).

Preformed cyclin E/ Cdk2 complex did not bind to AB or EF domain of AR, and neither cyclin D1 nor cyclin A bound to AR enough to be detectable (data not shown).

The deletion mutant proteins of AR (AB) were tested for transcription function in transient expression system and CAT assays in the absence or presence of an expression vector for cyclin E. In agreement with the results of *in vitro* binding activity assay (Fig. 4c) , cyclin E enhanced the transcription activities of N-terminally truncated mutant proteins of AR (AB) , such as del 62, del 72 and del82 , whereas the transcriptional activities of C-terminally truncated mutant proteins of AR (AB) , including del 13, del 14 and del 15, did not respond to cyclin E expression with increased CAT activity (closed bars in Fig. 4a). Especially when a region between amino acid 418 and 566 was deleted from AR(AB) (del 13) the ability of cyclin E to enhance its transcription was markedly attenuated (Fig. 4a) . These results indicate that the binding of cyclin E to the C-terminal portion (amino acids 418 to 566) of AR(AB) is indispensable for its ability to enhance AF-1 function of AR. Moreover, when both the cyclin E-binding region and the transactivation domain were deleted from full-length AR (del E in Fig. 4b), not only the transcriptional activation of AR by DHT but also the ability of cyclin E to enhance AR transcriptional activity in the presence of DHT was completely abolished (Fig. 4b).

### Industrial Applicability

The present invention revealed a novel action mechanism of anti-androgen agents through inhibition of the interaction of cyclin E with androgen receptor (AR). It providess methods of screening for anti-androgen agents based on this mechanism. Compounds of this invention that inhibit the interaction of cyclin E with androgen receptor (AR) is expected to repress the activation of androgen receptor (AR) which can be resistant to preexisting anti-androgen agents. Therefore, the present invention opens up novel possibilities for therapy using anti-androgen agents that had their limitations until now. Compounds of this invention that inhibit the interaction of cyclin E with androgen receptor (AR) is expected to be useful pharmaceutical compositions as therapeutic agents for diseases such as prostate cancer, benign prostatic hyperplasia, male pattern baldness, precocious puberty, acne vulgaris, seborrhea, and hypertrichosis. Furthermore, if these compounds are administered in advance, they are expected to prevent or delay the development of diseases, such as, prostate cancer, benign prostatic hyperplasia, male pattern baldness, precocious puberty, acne vulgaris, seborrhea, and hypertrichosis. Thus, these compounds are also expected to be preventive agents for the above diseases.

## Claims

1. A method of screening for a compound that inhibits the interaction of cyclin E with androgen receptor, comprising the steps of:
(a) contacting cyclin E and the androgen receptor in the presence of a test sample,
(b) detecting the binding of cyclin E to the androgen receptor, and
(c) selecting a compound that decreases the binding compared to when it is detected in the absence of the test sample.

2. A method of screening for a compound that inhibits interaction of cyclin E with androgen receptor, comprising the steps of:
(a) contacting a test sample with a cell into which a vector expressing cyclin E, a vector expressing androgen receptor, and a vector in which a reporter gene is operably linked to the downstream of androgen receptor responsive element, have been introduced,
(b) detecting the reporter activity in the cell, and,
(c) selecting a compound that decreases the reporter activity compared to when it is detected without contacting the test sample.

3. A compound that inhibits the interaction of cyclin E with androgen receptor, a pharmaceutically acceptable salt thereof, or a prodrug thereof.

4. The compound, pharmaceutically acceptable salt, or prodrug of claim 3, selected by the method of claim 1 or 2.

5. A pharmaceutical composition comprising the compound, pharmaceutically acceptable salt, or prodrug of claim 3 or 4 as the active ingredient.

6. The pharmaceutical composition of claim 5, which is an anti-androgen agent.

7. The pharmaceutical composition of claim 5 or 6, which is used for preventing and treating diseases selected from the group consisting of prostate cancer, benign prostatic hyperplasia, male pattern baldness, precocious puberty, acne vulgaris, seborrhea, and hypertrichosis.
